(19) Europäisches Patentamt
European Patent Office
Office européen des brevets

(11) Publication number: 0 271 909 A2

(12) **EUROPEAN PATENT APPLICATION**

(21) Application number: 87118724.1

(51) Int. Cl.[4]: A61K 31/23

(22) Date of filing: 17.12.87

(30) Priority: 17.12.86 JP 301961/86

(43) Date of publication of application: 22.06.88 Bulletin 88/25

(84) Designated Contracting States: BE CH DE ES FR GB LI NL SE

(71) Applicant: GREEN CROSS CORPORATION
15-1, Imabashi 1-chome Higashi-ku
Osaka-shi
Osaka(JP)

(72) Inventor: Inoue, Tadaaki
540-6, Oharano Haikata-cho Nishigyo-ku
Kyoto-shi Kyoto(JP)
Inventor: Sugio, Shigetoshi c/o Green Cross Corporation
Ryokufu-ryo 24-9, Kita Nakafuri 1-chome
Hirakata-shi Osaka(JP)
Inventor: Imagawa, Takashi
31-1, Hiyodoridai 2-chome Kita-ku
Kobe-shi Hyogo(JP)
Inventor: Okamoto, Hiroyuki
3-18-302, Nagate-cho 5-chome Nada-ku
Kobe-shi Hyogo(JP)

(74) Representative: Hansen, Bernd, Dr.rer.nat. et al
Hoffmann, Eitle & Partner Patentanwälte
Arabellastrasse 4
D-8000 München 81(DE)

(54) **Triglyceride composition.**

(57) A triglyceride composition comprising two or more triglycerides represented by the following general formula:

$$CH_2O-R_1$$
$$|$$
$$CHO-R_2$$
$$|$$
$$CH_2O-R_3$$

wherein $R_1$, $R_2$ and $R_3$, which may be the same or different, each represents an acyl group derived from an aliphatic acid having from 20 to 22 carbon atoms, an acyl group derived from an aliphatic acid having from 14 to 18 carbon atoms, or an acyl group derived from an aliphatic acid having from 6 to 12 carbon atoms, wherein the acyl group derived from an aliphatic acid having from 20 to 22 carbon atoms, the acyl group derived from an aliphatic acid having from 14 to 18 carbon atoms, and the acyl group derived from an aliphatic acid having from 6 to 12 carbon atoms are bound as $R_1$, $R_2$ and $R_3$ in such a manner that the aliphatic acids which are produced when all of the triglycerides of said composition are hydrolyzed consist essentially of 1 to 20 mol% of an aliphatic acid having 20 to 22 carbon atoms, 30 to 50 mol% of an aliphatic acid having 14 to 18 carbon atoms and 40 to 60 mol% of an aliphatic acid having from 6 to 12 carbon atoms, provided that the sum of the three aliphatic acids equals 100 mol%. The composition is useful as an active ingredient of nutrient infusions, and agents for preventing and treat-

EP 0 271 909 A2

ing arteriosclerosis or hyperlipemia.

# TRIGLYCERIDE COMPOSITION

## FIELD OF THE INVENTION

The present invention relates to a novel triglyceride composition.

## BACKGROUND OF THE INVENTION

Triglycerides, which consist of one molecule of glycerin combined with three molecules of an aliphatic acid by an ester linkage, are extensively used as lipid components in emulsions, nutrient infusions, edible fats and oils, etc. Commercially available fat emulsions for intravenous application (e.g. Intralipos®) are mainly composed of soybean oil which is a triglyceride containing long-chain and essential aliphatic acids as the fatty acid components. Fat emulsions for intravenous application are clinically used worldwide as nutrient infusions.

Attempts have been made to realize clinical use of lipid components that are composed of triglycerides of middle-chain aliphatic acids that are more rapidly metabolized than long-chain aliphatic acids and which serve as heat sources. Also, emulsions using lipid components that are mixtures of equal amounts of triglycerides of middle-chain aliphatic acids and those of long-chain aliphatic acids have been formulated in a dosage form (EP 71995A; Lipofunzin®, tradename for a product manufactured by Braun-Mersungen AG). It has also been attempted to prepare fat emulsions from triglycerides formed by effecting ester exchange between triglycerides of middle-chain aliphatic acids of vegetable oil origin and triglycerides of long-chain aliphatic acids (WO 86/01715).

Increasing attention is being drawn to the use as nutrient infusions of lipid compositions that are made either of triglycerides of middle chain aliphatic acids alone or mixtures thereof with triglycerides of long-chain aliphatic acids. However, these lipid compositions, as they are available today, are not completely effective in supplying nutrients, promoting the biosynthesis of proteins or in suppressing the decomposition of somatic proteins.

## SUMMARY OF THE INVENTION

An object, therefore, of the present invention is to provide a triglyceride composition that is effective in supplying nutrients, promoting the biosynthesis of proteins and in suppressing the decomposition of somatic proteins.

Another object of the present invention is to provide a triglyceride composition that is effective for the purpose of treating and preventing arteriosclerosis and hyperlipemia.

In order to attain these objects, a triglyceride composition has been synthesized in the present invention that contains in appropriate proportions not only a group derived from a long-chain aliphatic acid and a group derived from a middle-chain aliphatic acid but also a group derived from a long-chain aliphatic acid having 20 - 22 carbon atoms. It has been found in the present invention that this triglyceride composition is effective in supplying nutrients and suppressing the decomposition of somatic proteins and that it is also capable of effectively treating and preventing hyperlipemia.

More specifically, the present invention relates to a triglyceride composition comprising two or more triglycerides represented by the following general formula:

$$\begin{array}{l} CH_2O - R_1 \\ | \\ CHO - R_2 \\ | \\ CH_2O - R_3 \end{array}$$

wherein $R_1$, $R_2$ and $R_3$, which may be the same or different, each represents an acyl group derived from an aliphatic acid having from 20 to 22 carbon atoms, an acyl group derived from an aliphatic acid having from 14 to 18 carbon atoms, or an acyl group derived from an aliphatic acid having from 6 to 12 carbon atoms, wherein the acyl group derived from an aliphatic acid having from 20 to 22 carbon atoms, the acyl group derived from an aliphatic acid having from 14 to 18 carbon atoms, and the acyl group derived from an aliphatic acid having from 6 to 12 carbon atoms are bound as $R_1$, $R_2$ and $R_3$ in such a manner that the aliphatic acids which are produced when all of the triglycerides of said composition are hydrolyzed consist essentially of 1 to 20 mol% of an aliphatic acid having from 20 to 22 carbon atoms, 30 to 50 mol% of an aliphatic acid having form 14 to 18 carbon atoms and 40 to 60 mol% of an aliphatic acid having from 6 to 12 carbon atoms (the sum of the three aliphatic acids totaling 100 mol%).

## DETAILED DESCRIPTION OF THE INVENTION

The aliphatic acid components in the triglyceride composition of the present invention are an aliphatic acid having from 20 to 22 carbon

atoms, an aliphatic acid having from 14 to 18 carbon atoms, and an aliphatic acid having from 6 to 12 carbon atoms.

The aliphatic acid having from 20 to 22 carbon atoms may be saturated or unsaturated but it is preferably unsaturated. Specific examples of this aliphatic acid include arachidonic acid, eicosapentaenoic acid and docosahexaenoic acid. Aliphatic acids having from 20 to 22 carbon atoms may be used alone or in combinations of two or more. A particularly preferred example is eicosapentaenoic acid.

The aliphatic acid having from 14 to 18 carbon atoms may be saturated or unsaturated but it is preferably unsaturated. Specific examples of this aliphatic acid include myristic acid, palmitic acid, stearic acid, oleic acid, linoleic acid and linolenic acid. Aliphatic acids having from 14 to 18 carbon atoms may be used alone or in combinations of two or more. A particularly preferred example is linoleic acid.

The aliphatic acid having from 6 to 12 carbon atoms may be saturated or unsaturated but it is preferably saturated. It is also preferred to use aliphatic acids having from 8 to 10 carbon atoms. Specific examples of this aliphatic acid include caproic acid, caprylic acid, capric acid, and lauric acid. Aliphatic acids having from 6 to 12 carbon atoms may be used or in combinations of two or more. Particularly preferred examples are a combination of caprylic acid and capric acid.

The triglyceride composition of the present invention contains in specific proportions acyl groups derived from aliphatic acids having from 20 to 22 carbon atoms, 14 to 18 carbon atoms and 6 to 12 carbon atoms as $R_1$ , $R_2$ and $R_3$ of the general formula in the triglyceride components of said composition. The proportions of these acyl groups may be defined as follows: the acyl groups derived from aliphatic acids having from 20 to 22 carbon atoms, 14 to 18 carbon atoms and 6 to 12 carbon atoms are bound as $R_1$ , $R_2$ and $R_3$ in such a manner that when all of the aliphatic acids in the triglyceride composition are freed by subjecting the triglyceride composition to hydrolysis, said aliphatic acids consist essentially of 1 to 20 mol% of an aliphatic acid having from 20 to 22 carbon atoms, 30 to 50 mol% of an aliphatic acid having from 14 to 18 carbon atoms, and 40 to 60 mol% of an aliphatic acid having from 6 to 12 carbon atoms. In a preferred embodiment, the aliphatic acids consist of 10 to 30 mol% of caprylic acid, 20 to 40 mol% of capric acid (provided that the sum of caprylic acid and capric acid is 40 to 60 mol%), 30 to 50 mol% of linoleic acid, and 1 to 20 mol% of eicosapentaenoic acid.

An example. of the triglyceride composition that contains the acyl groups derived from the respective aliphatic acids in the proportions specified above is a mixture of triglycerides in which one trilglyceride is bound to two or more of the acyl groups in mixture that are derived from aliphatic acids having from 20 to 22 carbon atoms, 14 to 18 carbon atoms, and 6 to 12 carbon atoms, respectively, and in which said compositon taken as a whole is analyzed to contain 1 to 20 mol% of an aliphatic acid having from 20 to 22 carbon atoms, 30 to 50 mol% of an aliphatic acid having from 14 to 18 carbon atoms, and 40 to 60 mol% of an aliphatic acid having from 6 to 12 carbon atoms.

The method of producing the tryglyceride composition of the present invention is exemplified as described below.

The triglyceride composition of the present invention may be produced by esterifying the respective constituent aliphatic acids with glycerin in a conventional manner, as described in Advances in Lipid Research, 14 , 230 (1976), Academic press, New York. In this case, the aliphatic acids are preferably subjected to reaction in the form of their reactive derivatives such as acid halides and acid anhydrides. In this reaction, one mole of glycerin may be reacted with, for example, 2 to 3 moles of an aliphatic acid having from 6 to 12 carbon atoms, 2 to 3 moles of an aliphatic acid having from 14 to 18 carbon atoms, and 0.1 to 0.5 moles of an aliphatic acid having from 20 to 22 carbon atoms.

In a preferred embodiment, one mole of glycerin may be reacted with 1 to 15 moles of caprylic acid, 1 to 15 moles of capric acid, 2 to 3 moles of linoleic acid, and 0.1 to 0.5 moles of eicosapentaenoic acid. Solvents to be used are inert to the reaction and a preferred example is pyridine. The reaction is usually carried out for a period of from 10 to 60 minutes, preferably under refluxing conditions.

The triglyceride composition of the present invention is capable of promoting the biosynthesis of proteins, suppressing protein metabolism, preventing arteriosclerosis, and removing lipid components from the blood. Therefore, this composition is useful as an active ingredient of nutrient infusions, agents for preventing and treating arteriosclerosis, or agents for preventing and treating hyperlipemia. When human beings are put under stress as caused by traumas or burns, the release of free aliphatic acids from fat tissues, as well as the decomposition of glycogen in the liver and somatic proteins are accelerated, thereby causing increases in the concentrations of free aliphatic acids and glucose in blood. Under such conditions, the living body is unable to utilize glucose or free aliphatic acids as sources of energy. Therefore, the triglyceride composition of the present invention is advantageously used as a nutrient infusion into

patients suffering from stress.

The triglyceride composition of the present invention is advantageously administered parenterally, in particular, intravenously or rectally, in the form of a fat emulsion. A dose of 100 to 1,000 ml of a 10 w/v% of the fat emulsion is administered onece a day by intravenous drip. The dose is suitably adjusted in accordance with the body weight and the symptom. The triglyceride composition is administered intravenously in an amount of 2 g (20 ml of the emulsion) or less per day per kg of the body weight.

A fat emulsion may be prepared from the triglyceride composition by any of the known methods, as described in J. Amer. Oil. Chem. Soc., 32, 365-370 (1955).

The following examples are provided for the purpose of further illustrating the present inveniton but should not be taken as limiting.

EXAMPLE 1

1.41 g (4.6 mmol) of eicosapentaenoic acid was dissolved in 10 ml of benzene. To the solution, 1.64 g (13.8 mmol) of thionylchloride was added and the mixture was refluxed for 30 minutes in a nitrogen atmosphere. After completion of the reaction, the solvent was distilled off to obtain 1.45 g of eicosapentaenoic acid chloride.

By the same prodecures, chlorides of caprylic acid, capric acid and linoleic acid were obtained.

Next, 1.84 g (0.020 mmol) of concentrated glycerin was dissolved in 200 ml of pyridine. To the solution, 3.57 g (0.022 mmol) of caprylic acid chloride, 4.20 g (0.022 mmol) of capric acid chloride, 12.85 g (0.043 mmol) of linoleic acid chloride and 0.97 g (0.0030 mmol) of eicosapentaenoic acid chloride were added, and the mixture was refluxed for 30 minutes in a nitrogen atmosphere. Water was added to the reaction mixture and 19.2 g of a crude product was obtained by extraction with ethyl acetate. The crude product was purified by column chromatography (on silica gel; elution with a mixed solvent of petroleum ether and ethyl acetate in a mixing ratio of 95:5) to obtain a purified product in a yield of 6.03 g.

The results of infrared spectrum analysis is as follows. IR $\nu$: 720, 1150, 1460, 1750, 2850, 2900 $cm^{-1}$

From the absence of any absorption due to OH group in the neighborhood of 3500 $cm^{-1}$, the product of synthesis was found to be a triglyceride composition.

EXAMPLE 2

The triglyceride obtained in Example 1 was transmethylated with a 5 w/v% hydrochloric acid-methanol solution. The so treated triglyceride was analyzed for the composition of aliphatic acids by gas chromatography using a hydrogen flame ionization detector and a column packing material that consisted of the particles of diatomaceous earth (100 200 mesh) which were coated with 10 w/v% diethylene glycol succinate.

As a result, it was found that the triglyceride of Example 1 comprised 24.0 mol% of caprylic acid; 29.4 mol% of capric acid; 44.2 mol% of linoleic acid; and 2.4 mol% of eicosapentaenoic acid, The respective aliphatic acids were identified by comparison with authentic standards in terms of retention times.

EXAMPLE 3

A fat emulsion was prepared by homogenizing a formulation consisting of 10 w/v% of the triglyceride obtained in Example 1, 1.2 w/v% of purified phospholipid, 2.21 w/v% of concentrated glycerin and an appropriate amount of water. The emulsion was distributed among ampules and sterilized by autoclaving in a conventional manner to prepare injections.

While the invention has been described in detail and with reference to specific embodiments thereof, it will be apparent to one skilled in the art that various changes and modifications can be made therein without departing from the spirit and scope thereof.

**Claims**

1. A triglyceride composition comprising two or more triglycerides represented by the following general formula:

$$\begin{array}{l} CH_2O-R_1 \\ | \\ CHO-R_2 \\ | \\ CH_2O-R_3 \end{array}$$

wherein $R_1$, $R_2$ and $R_3$, which may be the same or different, each represents an acyl group derived from an aliphatic acid having from 20 to 22 carbon atoms, an acyl group derived from an aliphatic acid having from 14 to 18 carbon atoms, or an acyl group derived from an aliphatic acid having from 6

to 12 carbon atoms, wherein the acyl group derived from an aliphatic acid having from 20 to 22 carbon atoms, the acyl group derived from an aliphatic acid having from 14 to 18 carbon atoms, and the acyl group derived from an aliphatic acid having from 6 to 12 carbon atoms are bound as $R_1$ , $R_2$ and $R_3$ in such a manner that the aliphatic acids which are produced when all of the triglycerides of said composition are hydrolyzed consist essentially of 1 to 20 mol% of an aliphatic acid having 20 to 22 carbon atoms, 30 to 50 mol% of an aliphatic acid having 14 to 18 carbon atoms and 40 to 60 mol% of an aliphatic acid having from 6 to 12 carbon atoms, provided that the sum of the three aliphatic acids equals 100 mol%.

2. The triglyceride composition according to claim 1, wherein the aliphatic acid having from 20 to 22 carbon atoms is eicosapentaenoic acid.

3. The triglyceride composition according to claim 1, wherein the aliphatic acid having from 14 to 18 carbon atoms is linoleic acid.

4. The triglyceride composition according to claim 1, wherein the aliphatic acid having from 6 to 12 carbon atoms is selected from the group cosisting of caprylic acid and capric acid.

5. The triglyceride composition according to claim 1, which is prepared by subjecting glycerin and a mixture of aliphatic acids, corresponding to $R_1$ , $R_2$ and $R_3$ or reactive derivatives thereof to esterification.

6. The triglyceride composition according to claim 5, wherein the mixture of aliphatic acids, per one mole of glycerin, comprises 2 to 3 moles of an aliphatic acid having from 6 to 12 carbon atoms, 2 to 3 moles of an aliphatic acid having from 14 to 18 carbon atoms, and 0.1 to 0.5 moles of an aliphatic acid having from 20 to 22 carbon atoms.

7. The triglyceride composition according to claim 1, wherein said aliphatic acid having from 20 to 22 carbon atoms is an unsaturated aliphatic acid.

8. The triglyceride composition according to claim 1, wherein said aliphatic acid having from 14 to 18 carbon atoms is an unsaturated aliphatic acid.

9. The triglyceride composition according to claim 1, wherein said aliphatic acid having from 6 to 12 carbon atoms is a saturated fatty acid.

10. The triglyceride composition according to claim 1, wherein the aliphatic acids which are produced when all of the triglycerides of said composition are hydrolyzed consist essentially of 10 to 30 mol% of caprylic acid, 20 to 40 mol% of capric acid (provided that the sum of caprylic acid and capric acid is 40 to 60 mol%), 30 to 50 mol% of linoleic acid and 1 to 20 mol% of eicosapentaenoic acid.

11. The triglyceride composition according to claim 6, wherein the mixture of aliphatic acids, per one mole of glycerin, comprises 1 to 15 mols of caprylic acid, 1 to 15 mols of capric acid, 2 to 3 mols of linoleic acid and 0.1 to 0.5 mols of eicosapentaenoic acid.